# EUROPÄISCHE PATENTSCHRIFT

(11) **EP 1 600 439 B1**
(45) Veröffentlichungstag und Bekanntmachung des Hinweises auf die Patenterteilung: **03.02.2016**
(21) Anmeldenummer: 05010559.2
(22) Anmeldetag: 14.05.2005
(51) Int. Cl.: C07C 333/30

(54) **Herstellung von derivatisierten Dithiolen**
Preparation of dithiol derivatives
Préparation de dérivés de dithiols

(30) Priorität: 26.05.2004 DE 102004025730
(43) Veröffentlichungstag der Anmeldung: 30.11.2005
(73) Patentinhaber: LANXESS Deutschland GmbH, 50569 Köln (DE)
(72) Erfinder: Buding, Hartmuth, Dr., 52445 Titz (DE); Weidenhaupt, Hermann-Josef, Dr., 50529 Pulheim (DE); Jeske, Winfried, 51399 Burscheid (DE); Kleiner, Thomas, Dr., 51519 Odenthal (DE)

(56) Entgegenhaltungen:
- EP-A- 0 432 417
- EP-A- 0 530 590
- GB-A- 1 388 280
- US-A- 3 900 471

## Beschreibung

Die Erfindung betrifft ein Verfahren zur Herstellung von mit Dithiocarbamidsäuren derivatisierten Dithiolen.

Die Herstellung von mit Dithiocarbamidsäuren derivatisierten Dithiolen ist prinzipiell bekannt (vgl. z.B. DE-A 22 56 511 und EP-A 530 590).

Die DE-A 22 56 511 offenbart u.a. die Synthese von mit Dithiocarbamidsäuren derivatisierten Dithiolen über Bunte-Salze. Auf Seite 39, 3. Absatz, wird eine derartige Synthese am Beispiel der Verbindung 1,4-Bis(hexahydro-1H-azepin-1-ylthiocarbonyl-dithio)-2-buten wie folgt beschrieben: Man setzt 0,1 mol 1,4-Dichlorbuten-2 in wässrigem Ethanol mit Natriumthiosulfatpentahydrat bei Siedetemperatur um und entfernt anschließend durch Destillation das Ethanol. Sodann fügt man zu der wässrigen Bunte-Salzlösung weiteres Wasser, 0,3 mol Natriumacetattrihydrat und ca. 0,23 mol Formaldehyd zu. Zu dieser Mischung tropft man dann bei Raumtemperatur während 0,5 h eine wässrige Lösung von Natriumhexahydro-1H-azepin-1-ylthiocarbothiolat zu und rührt den Ansatz noch für weitere 1,5 h bei Raumtemperatur nach. Ein organisches Lösungsmittel ist bei der Umsetzung nicht zugegen. Das ölige Reaktionsprodukt wird am Ende der Umsetzung mit Chloroform extrahiert, die organische Phase mit Wasser gewaschen und über wasserfreiem Natriumsulfat getrocknet. Zur Gewinnung des 1,4-Bis(hexahydro-1H-azepin-1-ylthiocarbonyldithio)-2-butens wird das Chloroform verdampft. Nachteil dieses Verfahrens ist einmal die hohe Abwasserbelastung durch Natriumacetat, d.h. das Abwasser hat aufgrund der hohen Konzentration an Natriumacetat einen hohen chemischen Sauerstoffbedarf (CSB). So werden in dem vorbenannten Beispiel bei der angegebenen Ausbeute von 71% für 1 mol hergestelltes Produkt ca. 4,2 mol Natriumacetat eingesetzt, was aus ökologischer Sicht ein sehr ungünstiges Verhältnis darstellt. Bei der Nacharbeitung der allgemeinen Hinweise der DE-A 22 56 511 ausgehend von 1,6-Dichlorhexan in Bezug auf die erfindungsgemäßen Verbindungen der Formel (I), die nachstehend näher erläutert sind, wurden zum anderen schwer filtrierbare und schwer waschbare Produkte in unzureichender Ausbeute und teilweise unzureichender Reinheit (Wirkstoffgehalt) erhalten (vgl. Beispiele 17 und 18).

Die EP-A 530 590 verweist hinsichtlich der Herstellung von mit Dithiocarbamidsäuren derivatisierten Dithiolen z.B. auf die EP-A 432 417. Die EP-A 432 417 offenbart als Syntheseweg für die Herstellung von mit Dibenzyldithiocarbamidsäure derivatisierten Dithiolen in sehr allgemeiner Weise die Umsetzung von 1,2-Dichlorethan mit Natriumthiosulfat in wässriger Lösung und anschließender Umsetzung des entstandenen Bis-Bunte-Salzes mit Natriumdibenzyldithiocarbamat (Seite 4, Zeilen 50 bis 56). Über vorteilhaft anzuwendende Reaktionsparameter werden keine Angaben gemacht. Auch werden keine Ausführungsbeispiele für die Synthese angegeben. Eigene Versuche zur Nacharbeitung des allgemeinen Synthesehinweises der EP-A 432 417 am Beispiel von 1,6-Dichlorhexan bezüglich der erfindungsgemäßen Verbindungen der Formel (I), die nachstehend näher erläutert sind, haben schwer filtrierbare und schwer waschbare Produkte in unzureichender Ausbeute und teilweise auch unzureichender Reinheit (Wirkstoffgehalt) ergeben (vgl. Beispiele 19 und 20).

Über ein technisch einfaches, umweltschonendes und gut praktizierbares Herstellverfahren, das die erfindungsgemäßen Verbindungen der Formel (I) in hohen Ausbeuten und in hohen Reinheiten liefert, ist nichts beschrieben.

Aufgabe der vorliegenden Erfindung ist es, ein technisch einfaches, wirtschaftliches und möglichst umweltschonendes und gut praktizierbares Herstellverfahren für die Verbindungen der Formel (I) bereitzustellen, das Produkte in hoher Ausbeute und Reinheit liefert.

Mit den Herstellverfahren nach Stand der Technik kann das gesetzte Ziel nicht erreicht werden.

Gegenstand der Erfindung ist daher ein Verfahren zur Herstellung von Verbindungen der Formel (I)

R¹R² N-(C=S)-S-S-(CH₂)ₙ-S-S-(C=S)-N R¹R² (I),

worin die Reste R¹ und R² unabhängig voneinander Wasserstoff, Alkyl, Cycloalkyl, Aralkyl, Phenyl oder substituiertes Phenyl, oder Alkylaryl bedeuten oder zusammen mit dem jeweiligen Stickstoffatom einen heterocyclischen Ring mit 4 bis 8 Kohlenstoffatomen bilden und n eine ganze Zahl von 2 bis 8 darstellt,
das dadurch gekennzeichnet ist, dass man die Verbindungen der Formel (II)

[R¹R² N-(C=S)-S]_{z}Me¹ (II),

worin die Reste R¹ und R² die gleiche Bedeutung wie in der Formel (I) haben und z = 1 ist, wenn Me¹ ein Alkalimetall- oder ein Ammoniumion bedeutet und z = 2 ist, wenn Me¹ ein Erdalkalimetallion bedeutet,
mit den Verbindungen der Formel (III)

Me²O₃SS-(CH₂)ₙ-SSO₃Me³ (III),

worin Me² und Me³ gleich oder verschieden sind und einwertige Metallionen oder Ammoniumionen bedeuten und n die gleiche Bedeutung wie in der Formel (I) hat,

in Gegenwart von Wasser, Carbonylverbindungen und organischen Lösungsmitteln in einem pH-Wert-Bereich von 7 bis 14 bei einer Reaktionstemperatur von 0° bis 90°C bis zu einer Ausbeute von mindestens 80 % der Theorie, bezogen auf die Verbindungen der Formel (III), umsetzt, wobei die Verbindungen der Formel (II) in Mengen von 180 bis 250 Mol-%, bezogen auf die Mole an eingesetzten Verbindungen der Formel (III), die Carbonylverbindungen in Mengen von 5 bis 600 Mol-%, bezogen auf die Mole an eingesetzten Verbindungen der Formel (III), und die organischen Lösungsmittel in Mengen von 2 bis 100000 Gew.-Teilen, bezogen auf 100 Gew.-Teile der theoretisch zu erwartenden Menge an Verbindungen der Formel (I), eingesetzt werden.

Die Reste R¹ und R² in den Formeln (I) und (II) stehen bevorzugt für Wasserstoff, Alkyl, geradkettig oder verzweigt mit 3 bis 24 Kohlenstoffatomen, Cycloalkyl mit 3 bis 8 Kohlenstoffatomen, Aralkyl mit 7 bis 26 Kohlenstoffatomen, Phenyl, gegebenenfalls substituiert mit Halogenatomen, Nitro- und/oder OH-Gruppen, oder Alkylaryl mit 7 bis 26 Kohlenstoffatomen oder bilden zusammen mit dem jeweiligen Stickstoffatom einen heterocyclischen Ring mit 4 bis 8 Kohlenstoffatomen.

Beispiele für Alkylreste sind Methyl, Ethyl, Propyl, i-Propyl, n-Butyl, i-Butyl, sec.-Butyl, tert.-Butyl, n-Amyl, Hexyl, n-Octyl, 2-Ethylhexyl, Decyl, Dodecyl, Tetradecyl, Hexadecyl, Octadecyl, Eicosyl oder Docosyl. Beispiele für Cycloalkylreste sind Cyclopropyl, Cyclopentyl, Methylcyclopentyl, Cyclohexyl, Methylcyclohexyl, Cycloheptyl oder Cyclooctyl. Beispiele für Aralkylreste sind Benzyl oder Phenylethyl. Beispiele für Alkylarylreste sind Tolyl oder Xylyl.

Beispiele für die Aminoreste R¹ R² N in den Formeln (I) und (II) können formal von den Aminen Dimethylamin, Diethylamin, Dipropylamin, Di-i-propylamin, Di-n-Butylamin, Di-i-Butylamin, Ditert. Butylamin, Dioctylamin, Di-2-ethylhexylamin, Dinonylamin, Dicyclohexylamin, Diphenylamin, Dibenzylamin, Methylphenylamin, Ethylphenylamin, Phenylcyclohexylamin, Phenylbenzylamin oder Benzylcyclohexylamin oder von sogenannten technischen Fettaminen, wie z.B. Kokosamin, Talgamin oder Oleylamin (vgl. hierzu Ullmanns Encyklopädie der technischen Chemie, S. 448, Verlag Chemie, 1976), oder von den cyclischen Aminen Pyrrolidin, 2,5-Dimethylpyrrolidin, Piperidin, 2-Methylpiperidin, Morpholin, 2,6-Dimethylmorpholin, Hexahydro-1H-azepin, Hexahydro-1-(2H)-azocin oder Octahydro-1H-azonin abgeleitet werden.

Die bevorzugten Aminoreste R¹ R² N in den Formeln (I) und (II) können formal von den Fettaminen Kokosamin, Talgamin oder Oleylamin, oder Gemische hieraus, oder von Dicyclohexylamin oder Dibenzylamin abgeleitet werden, ganz besonders bevorzugt von Dibenzylamin.

Die Laufzahl n in der Formel (I) stellt eine ganze Zahl bevorzugt von 4 bis 8, dar. Die Laufzahl n = 6 ist ganz besonders bevorzugt.

In den Verbindungen der Formel (II) hat Me¹ die Bedeutung von Erdalkalimetallionen, wenn z = 2 ist, und die Bedeutung von Alkalimetall- oder Ammoniumionen, bevorzugt von Alkalimetallionen, wenn z = 1 ist. Aus der Reihe der Erdalkalimetallionen sind Calcium- und Bariumionen bevorzugt, ganz besonders bevorzugt sind Bariumionen. Aus der Reihe der Alkalimetallionen sind Kalium- und Natriumionen bevorzugt, ganz besonders bevorzugt sind Natriumionen.

Die Verbindungen der Formel (II) werden für das erfindungsgemäße Verfahren üblicherweise in einer Menge von ca. 180 bis 250 Mol-%, bevorzugt von 190 bis 240 Mol-%, ganz besonders bevorzugt von 200 bis 230 Mol-%, bezogen auf die Mole an eingesetzten Verbindungen der Formel (III), eingesetzt.

Die Herstellung der Verbindungen der Formel (II) aus Ammoniak bzw. Aminen und Schwefelkohlenstoff ist prinzipiell bekannt (vgl. z.B. Houben-Weyl, Methoden der organischen Chemie, Bd. 9, Seite 823ff, Georg Thieme Verlag, Stuttgart (1955); Ullmanns Encyklopädie der technischen Chemie, 4. neubearbeitete und erweiterte Auflage, Bd. 21, S. 89ff, Verlag Chemie, Weinheim (1982); DE-OS 22 56 511, Seite 38).

Die Herstellung der Verbindungen der Formel (III) ist z.B. aus den Patentveröffentlichungen EP-A 70 143, DE-A 22 56 511, EP-A 385 972 und EP-A 432 417 prinzipiell bekannt.

Für das erfindungsgemäße Herstellverfahren können die Verbindungen der Formel (II) und (III) (z.B. Duralink^{®} HTS von Firma Flexsys/Belgien) als isolierte Salze oder auch in Form ihrer Reaktionslösungen eingesetzt werden. Erforderlichenfalls sind die nach Stand der Technik hergestellten Verbindungen der Formeln (II) und (III) vor Verwendung für das erfindungsgemäße Verfahren in geeigneter Weise zu reinigen.

Nach einem neuen Herstellverfahren (vgl. Deutsche Patentanmeldung mit der Anmeldenummer 102004018193) können die Verbindungen der Formel (III) für das erfindungsgemäße Verfahren vorteilhaft direkt in Form ihrer Reaktionslösungen ohne weitere Reinigung eingesetzt werden. Bei diesem neuen Herstellverfahren für die Verbindungen der Formel (III) werden die zugrunde liegenden α,ω-Dihalogenalkane mit Thiosulfationen bei einer Reaktionstemperatur von ca. 80° bis 150°C in Wasser ohne Zusatz von Alkoholen und/oder Glykolen in einem pH-Wert-Bereich von 3 bis 9,8 umgesetzt.

Die Laufzahl n in der Formel (III) hat die gleiche Bedeutung wie in der Formel (I). Me² und Me³ sind in der Formel (III) gleich oder verschieden und bedeuten einwertige Metallionen oder Ammoniumionen. Bevorzugt sind Me² und Me³ gleich und bedeuten Alkalimetall- oder Ammoniumionen, bevorzugt Alkalimetallionen. Von den Alkalimetallionen sind Natrium- und Kaliumionen bevorzugt, ganz besonders bevorzugt sind Natriumionen.

Als Carbonylverbindungen für das erfindungsgemäße Verfahren kommen ganz oder nur teilweise in Wasser lösliche Aldehyde und/oder Ketone in Frage. Aus der Reihe der Aldehyde kommen z.B. in Frage Formaldehyd, Acetaldehyd oder Propionaldehyd, bevorzugt Formaldehyd. Selbstverständlich können von den Aldehyden auch beliebige Mischungen eingesetzt werden. Aus der Reihe der Ketone kommen z.B. in Frage Aceton, Diethylketon, Methylethylketon oder Methylpropylketon, bevorzugt Methylethylketon. Selbstverständlich können von den Ketonen auch beliebige Mischungen eingesetzt werden.

Von den erfindungsgemäßen Aldehyden und Ketonen können ebenfalls beliebige Mischungen untereinander zum Einsatz kommen.

Sofern der Dampfdruck der erfindungsgemäßen Aldehyde und/oder Ketone bei der erfindungsgemäßen Reaktionstemperatur hoch ist, ist es vorteilhaft, in einem Autoklaven zu arbeiten, damit das Reaktionsgemisch nicht an diesen Carbonylverbindungen durch Verdampfung verarmt.

Die erfindungsgemäßen Carbonylverbindungen werden als solche, d.h. gasförmig oder flüssig oder als handelsübliche, wässrige Lösung eingesetzt (vgl. Ullmanns Encyclopädie der technischen Chemie, 4. neubearbeitete und erweiterte Auflage, Bd. 11, S. 696, Verlag Chemie, Weinheim (1976)).

Von den erfindungsgemäßen Carbonylverbindungen ist für das erfindungsgemäße Herstellverfahren Formaldehyd ganz besonders bevorzugt, insbesondere in Form seiner wässrigen Lösung.

Für das erfindungsgemäße Verfahren kommen als organische Lösungsmittel aus der Reihe der aromatischen Lösungsmittel Benzol, Toluol, Xylol, Ethyl- oder Diethylbenzol, Tetralin, Chlor- oder Dichlorbenzol oder Anisol in Frage. Selbstverständlich können auch beliebige Mischungen aus den aromatischen Lösungsmitteln eingesetzt werden.

Aus der Reihe der aliphatischen Lösungsmittel kommen geradkettige oder verzweigte aliphatische Kohlenwasserstoffe mit 5 bis 12 Kohlenstoffatomen, wie z.B. Pentan, Hexan, Heptan oder Octan, oder Mischungen hieraus, in Betracht. Es können aber auch Kohlenwasserstoffgemische, die überwiegend aus aliphatischen Kohlenwasserstoffen bestehen, wie z.B. Petrolether mit einem Siedebereich von ca. 40° bis 70°C, Leichtbenzin mit einem Siedebereich von ca. 70° bis 90°C oder Mittelbenzin mit einem Siedebereich von ca. 90° bis 180°C, verwendet werden.

Aus der Reihe der cycloaliphatischen Lösungsmitteln kommen cycloaliphatische Kohlenwasserstoffe mit 5 bis 10 Kohlenstoffatomen, wie z.B. Cyclopentan, Methylcyclopentan, Cyclohexan, Methylcyclohexan, Cycloheptan oder Dekalin in Frage. Selbstverständlich können auch beliebige Mischungen aus den cycloaliphatischen Kohlenwasserstoffen eingesetzt werden.

Aus der Reihe der Ether kommen Diethylether, Di-i-propylether, Di-n-butylether oder Di-sec.-butylether, Methyl-tert.-butylether oder Gemische hieraus in Frage.

Gemäß der Erfindung können die Verbindungen der Formel (III), welche ganz oder teilweise in Wasser gelöst sind, und die Salze der Formel (II), welche ebenfalls ganz oder teilweise in Wasser gelöst sind, in einem kontinuierlichen Prozess miteinander umgesetzt werden, d.h. die beiden Edukte und die erfindungsgemäßen Ingredientien werden fortlaufend in ein Reaktionsrohr oder in eine 1-Kessel- oder Mehrkesselkaskade eingebracht und umgesetzt. Bevorzugt werden die Verbindungen der Formel (III), welche ganz oder teilweise in Wasser gelöst sind, in einem Reaktionsgefäß zusammen mit den erfindungsgemäßen Ingredientien vorgelegt und die Verbindungen der Formel (II), in fester Form oder ganz oder teilweise in Wasser gelöst, kontinuierlich oder portionsweise zugefügt. Die Geschwindigkeit der Zugabe der Verbindungen der Formel (II) zu den vorgelegten Verbindungen der Formel (III) ist abhängig von der gewählten Reaktionstemperatur, von der gewählten Ansatzgröße und von der zur Verfügung stehenden Kühlkapazität des Reaktionsgefäßes und kann durch Vorversuche leicht herausgefunden werden. Wenn viel Reaktionswärme durch Kühlung abgeführt werden kann, so kann eine höhere Dosiergeschwindigkeit gewählt werden als wenn nur wenig Reaktionswärme abgeführt werden kann. Typische Zudosierzeiten für die in Wasser z.B. vollständig gelösten Verbindungen der Formel (II) in die wässrige und organische Lösungsmittel enthaltende Vorlage mit den ganz oder teilweise gelösten Verbindungen der Formel (III) sind z.B. 0,25 bis 5 h, bevorzugt 0,5 bis 3 h. Für noch längere Zudosierzeiten besteht technisch keine Limitierung, jedoch dürften diese im Allgemeinen nicht wirtschaftlich sein. Selbstverständlich kann auch umgekehrt verfahren werden, d.h. die Verbindungen der Formel (III) können in fester Form oder ganz oder teilweise in Wasser gelöst zu den zusammen mit den erfindungsgemäßen Ingredientien vorgelegten Verbindungen der Formel (II), die ganz oder teilweise in Wasser gelöst sind, kontinuierlich oder portionsweise gegeben werden.

Bevorzugt wird das Edukt, welches zum vorgelegten Edukt hinzugefügt wird, in vollständig gelöster Form zugesetzt.

Die erfindungsgemäßen Carbonylverbindungen werden in Mengen von ca. 5 bis 600 Mol-%, bezogen auf die Mole an eingesetzten Verbindungen der Formel (III), eingesetzt. Die günstigste Menge an Carbonylverbindungen kann leicht durch Vorversuche ermittelt werden. Beim bevorzugten Herstellverfahren, bei dem die Verbindungen der Formel (II) zu den in der Vorlage befindlichen Verbindungen der Formel (III) hinzugefügt werden, werden bei langen Reaktionszeiten, d.h. bei Nachreaktionszeiten von mehr als 10 h, gerechnet ab Ende der Zugabe der wässrigen Lösungen der Verbindungen der Formel (II), ca. 5 bis 300 Mol-%, bevorzugt 10 bis 200 Mol-%, an Carbonylverbindungen, bezogen auf die Mole an eingesetzten Verbindungen der Formel (III), eingesetzt. Bei kürzeren Reaktionszeiten, d.h. bei Nachreaktionszeiten von etwa 0,5 bis etwa 10 h, gerechnet ab Ende der Zugabe der wässrigen Lösungen der Verbindungen der Formel (II), werden 25 bis 600 Mol-%, bevorzugt 37 bis 550 Mol-%, ganz besonders bevorzugt 50 bis 500 Mol-%, an Carbonylverbindungen, bezogen auf die Mole an eingesetzten Verbindungen der Formel (III), eingesetzt.

Die Löslichkeit der Verbindungen der Formel (III) in Wasser ist abhängig von der Art der Kationen Me² und Me³, von der Laufzahl n und von der Temperatur des Lösungsmittels Wasser. Die Löslichkeit dieser Verbindungen bei der gewählten Wassertemperatur kann durch Versuche leicht herausgefunden werden. Mit steigender Temperatur nimmt die Löslichkeit der Verbindungen der Formel (III) in Wasser im Allgemeinen zu. Im Falle von 1,6-Hexamethylen-bis-(Natriumthiosulfat)-Dihydrat sind bei ca. 70°C noch ca. 51 %ige wässrige Lösungen herstellbar.

Die Löslichkeit der Verbindungen der Formel (II) in Wasser ist abhängig von der Resten R¹ und R², von der Art des Kations Me¹ und von der Temperatur des Lösungsmittels Wasser. Die Löslichkeit dieser Verbindungen bei der gewählten Wassertemperatur kann durch Versuche leicht herausgefunden werden. Mit steigender Temperatur nimmt die Löslichkeit der Verbindungen der Formel (II) in Wasser im Allgemeinen zu. Im Falle von Natriumdibenzyldithiocarbamat sind bei ca. 20°C ca. 17 bis 21%ige wässrige Lösungen leicht herstellbar.

Die Anwendung von wässrigen Lösungen der Verbindungen der Formel (II) und (III) in sehr niedriger Konzentration ist technisch nicht limitiert, sondern nur unwirtschaftlich für das erfindungsgemäße Verfahren und aus Sicht möglichst kleiner Abwassermengen nicht vorteilhaft.

Das erfindungsgemäße Verfahren wird bei einer Reaktionstemperatur von ca. 0° bis 90°C, bevorzugt von 5° bis 85°C, ganz besonders bevorzugt von 10° bis 80°C, und in einem pH-Wert-Bereich von ca. 7 bis 14, bevorzugt von 8 bis 14, ganz besonders bevorzugt von 8,5 bis 13,5 bis zu einer Ausbeute von mindestens 80% der Theorie, bevorzugt von mindestens 85% der Theorie, bezogen auf die eingesetzten Verbindungen der Formel (III), durchgeführt.

Die pH-Werte im erfindungsgemäßen pH-Wert-Bereich werden je nach vorliegender pH-Wert-Situation vorzugsweise mit Säuren, bevorzugt mit anorganischen Säuren, wie z.B. Chlorwasserstoffsäure oder Schwefelsäure, oder Gemische hieraus, oder mit Basen, bevorzugt mit anorganischen Basen, wie z.B. Natronlauge oder Kalilauge, oder Gemische hieraus, eingestellt und kontrolliert. Abhängig vom pH-Wert der in Wasser gelösten Verbindungen der Formel (II) und (III) ist unter Umständen eine spezielle Einstellung für den erfindungsgemäßen pH-Wert-Bereich nicht nötig, da sich dieser von selbst einstellt (vgl. Beispiel 2). Selbstverständlich kann im Rahmen der vorliegenden Erfindung mit der Umsetzung der Verbindungen der Formel (III), welche sich nach dem bevorzugten Herstellverfahren in der Vorlage befinden, mit den Verbindungen der Formel (II), welche sich im Zulauf befinden, auch bei niedrigerem pH-Wert als 7 begonnen werden, wobei es dann aber darauf ankommt, dass mit der Zugabe der alkalisch reagierenden Verbindungen der Formel (II) in die Vorlage mit den Verbindungen der Formel (III) der pH-Wert des wässrigen Reaktionsgemisches sehr rasch in den bevorzugten pH-Wertbereich von 8 bis 14 ansteigt, andernfalls sind diese pH-Wert-Bereiche durch Zugabe von anorganischen Säuren bzw. Basen entsprechend einzustellen. Vorzugsweise sollte die Untergrenze des bevorzugten pH-Wert-Bereichs von 8 nach Zugabe von höchstens 20 Gew.-Teilen, bevorzugt von höchstens 10 Gew.-Teilen, bezogen auf 100 Gew.-Teilen der erfindungsgemäß einzusetzenden Menge an Verbindungen der Formel (II), erreicht werden. Nach der Zugabe der Verbindungen der Formel (II) zu den Verbindungen der Formel (III) führt man die Reaktion vorzugsweise im optimalen pH-Wert-Bereich von ca. 9 bis 13 zu Ende.

In einer bevorzugten Ausführungsform der Erfindung wird der optimale pH-Wert-Bereich von ca. 9 bis 13 vorteilhaft mit Kalium- oder Natriumhydrogencarbonat, bevorzugt mit Natriumhydrogencarbonat eingestellt. Die Menge an einzusetzendem Kalium- bzw. Natriumhydrogencarbonat beträgt ca. 1 bis 300 Gew.-Teile, bevorzugt 25 bis 200 Gew.-Teile, ganz besonders bevorzugt 50 bis 150 Gew.-Teile, bezogen auf 100 Gew.-Teile der theoretisch zu erwartenden Menge an Verbindungen der Formel (I). Die erfindungsgemäße Menge an Alkalihydrogencarbonat kann auf einmal unmittelbar vor Beginn der Reaktion zusammen mit den wässrigen Lösungen/Dispersionen an Verbindungen der Formel (III) in fester Form oder auch als wässrige Lösung vorgelegt werden oder während der Reaktion portionsweise oder auch kontinuierlich in fester Form oder auch als wässrige Lösung der Reaktionsmischung zugesetzt werden. Bevorzugt wird die erfindungsgemäße Menge an Alkalihydrogencarbonat zur Einstellung des optimalen pH-Wert-Bereichs auf einmal, unmittelbar vor dem Zulauf der wässrigen Lösungen der Verbindungen der Formel (II), in der Vorlage mit den erfindungsgemäßen Ingredientien und Verbindungen der Formel (III) hinzugefügt. Hierbei stellt sich üblicherweise ein pH-Wert im Bereich von ca. 7 bis 9 ein. Während des Zulaufs der Verbindungen der Formel (II) steigt der pH-Wert des Reaktionsgemisches rasch an und liegt dann nach Beendigung des Zulaufs, einschließlich der sich anschließenden Nachrührzeit, im optimalen pH-Wert-Bereich von ca. 9 bis 13.

Für das erfindungsgemäße Herstellverfahren werden die organischen Lösungsmittel in Mengen von ca. 2 bis 100000 Gew.-Teilen, bevorzugt von 3 bis 10000 Gew.-Teilen, ganz besonders bevorzugt von 4 bis 2000 Gew.-Teilen, bezogen auf 100 Gew.-Teile der theoretisch zu erwartenden Mengen an Verbindungen der Formel (I), eingesetzt. Hinsichtlich dieser Mengen an Lösungsmittel gibt es zwei Verfahrensvarianten. Bei der ersten Verfahrensvariante wird nur wenig Lösungsmittel eingesetzt, d.h. die Lösungsmittelmenge ist so klein, dass nur ein untergeordneter Anteil der insgesamt sich bildenden Menge an Verbindungen der Formel (I) im gewählten Lösungsmittel unter Reaktionsbedingungen gelöst wird. Hierbei handelt es sich um das sogenannte Slurry-Verfahren, d.h. man hat insbesondere am Ende der Umsetzung eine Feststoffphase neben flüssigen Phasen vorliegen. Bei der zweiten Verfahrensvariante wird viel Lösungsmittel eingesetzt, d.h. die Lösungsmittelmenge ist so groß, dass die gesamte sich bildende Menge an Verbindungen der Formel (I) unter Reaktionsbedingungen im gewählten Lösungsmittel gelöst wird. Hierbei handelt es sich um das sogenannte Lösungs-Verfahren, d.h. man hat während und am Ende der Umsetzung nur flüssige Phasen vorliegen. Die beiden Varianten sind nachstehend näher erläutert.

Beim Slurry-Verfahren werden die erfindungsgemäßen organischen Lösungsmittel in Mengen von ca. 2 bis 100 Gew.-Teilen, bevorzugt von 3 bis 95 Gew.-Teilen, ganz besonders bevorzugt von 4 bis 90 Gew.-Teilen, bezogen auf 100 Gew.-Teile der theoretisch zu erwartenden Menge an Verbindungen der Formel (I), eingesetzt.

Die günstigste Menge an Lösungsmittel für das Slurry-Verfahren kann leicht durch Vorversuche ermittelt werden. Sie ist abhängig von der Art des Lösungsmittels und der Reaktionstemperatur so zu wählen, dass am Ende der Reaktion nur geringe Mengen an den Verbindungen der Formel (I) im organischen Lösungsmittel gelöst sind. Die Hauptmenge an den Verbindungen der Formel (I) soll nach deren Bildung ungelöst, d.h. als Feststoff in den flüssigen Phasen vorliegen. Im Sinne der Erfindung soll beim Slurry-Verfahren die am Ende der Reaktion im organischen Lösungsmittel gelöste Menge an den Verbindungen der Formel (I) höchstens 15 Gew.-Teile, bevorzugt höchstens 10 Gew.-Teile, bezogen auf 100 Gew.-Teile durch Fest-Flüssig-Trennung (z.B. durch Filtration, Nutschen oder Zentrifugation) isolierbarem Produkt, betragen.

Beim Slurry-Verfahren ist es günstig, wenn sich eine kleine Menge der Verbindungen der Formel (I) im gewählten Lösungsmittel unter Reaktionsbedingungen löst. Nach unseren Abschätzungen liegt diese Menge bei mindestens 0,01 Gew.-Teilen, bevorzugt bei mindestens 0,1 Gew.-Teilen, bezogen auf 100 Gew.-Teile durch Fest-Flüssig-Trennung isolierbarem Produkt.

Für das Slurry-Verfahren ist als Lösungsmittel Toluol, Hexan, Leichtbenzin mit einem Siedebereich von ca. 70 bis 90°C, Cyclohexan oder Methyl-tert.-Butylether bevorzugt. Von diesen Lösungsmitteln kommen aber auch Mischungen untereinander als Prozesslösungsmittel in Frage. Ganz besonders bevorzugt ist Toluol als Lösungsmittel.

Beim Slurry-Verfahren liegt die erfindungsgemäße Reaktionstemperatur in einem ausgewählten Temperaturbereich von ca. 0° bis 60°C, bevorzugt von 5° bis 55°C, ganz besonders bevorzugt von 10° bis 50°C.

Die Abtrennung der ausgefallenen Verbindungen der Formel (I) beim Slurry-Verfahren erfolgt bevorzugt durch Fest-Flüssig-Trennung, wie z.B. durch Filtration (z.B. Filterpresse), durch Zentrifugation oder durch Nutschen. Nach der Abtrennung der festen Verbindungen der Formel (I) von der Mutterlauge werden diese in geeigneter Weise mit entionisiertem Wasser gewaschen und getrocknet.

Zur Verwendung der Verbindungen der Formel (I) als Vulkanisationsmittel für Dien-Kautschuk werden diese typischerweise gemahlen und gegebenenfalls mit einem geeignetem Öl, z.B. Mineralöl, zur Staubunterdrückung beaufschlagt, welches keinen negativen Einfluss auf die anwendungstechnischen Eigenschaften der Verbindungen der Formel (I) hat.

Beim Lösungs-Verfahren hingegen werden die erfindungsgemäßen organischen Lösungsmittel in Mengen von ca. 105 bis 100 000 Gew.-Teilen, bevorzugt von 120 bis 10 000 Gew.-Teilen, ganz besonders bevorzugt von 150 bis 2 000 Gew.-Teilen, bezogen auf 100 Gew.-Teile der theoretisch zu erwartenden Menge an Verbindungen der Formel (I), eingesetzt.

Die für das erfindungsgemäße Lösungs-Verfahren mindestens erforderliche Menge an Lösungsmittel kann leicht durch Vorversuche ermittelt werden. Sie ist abhängig von der Art des Lösungsmittels und der Reaktionstemperatur so zu wählen, dass am Ende der Reaktion die gebildete Mengen an Verbindungen der Formel (I) im organischen Lösungsmittel vollständig gelöst ist.

Für das Lösungs-Verfahren ist als Lösungsmittel Toluol, Xylol oder Chlorbenzol bevorzugt. Es können aber auch Mischungen dieser Lösungsmittel untereinander als Prozesslösungsmittel eingesetzt werden. Ganz besonders bevorzugt ist Toluol oder Chlorbenzol als Lösungsmittel.

Beim Lösungs-Verfahren liegt die erfindungsgemäße Reaktionstemperatur im Temperaturbereich von ca. 0° bis 90°C, bevorzugt von 5° bis 85°C, ganz besonders bevorzugt von 10° bis 80°C.

Nach beendeter Reaktion wird beim Lösungsverfahren die organische Phase von der wässrigen Phase abgetrennt. Gewünschtenfalls kann die separierte organische Phase mit Wasser gewaschen werden. Zur Gewinnung der Verbindungen der Formel (I) kann das organische Prozesslösungsmittel verdampft werden, vorzugsweise im Vakuum, und dann das erhaltene zähe Öl zur Kristallisation gebracht werden, was in der Regel sehr schwierig und zeitraubend ist. Sodann können die Verbindungen der Formel (I) wie beim Slurry-Verfahren gemahlen und zur Staubunterdrückung mit einem geeigneten Öl beaufschlagt werden. Um die aufwendigen Prozess-Schritte Mahlung und Beölung zu umgehen, können die organischen Lösungen, bestehend aus Prozesslösungsmittel und den darin gelösten Verbindungen der Formel (I), vorteilhaft mit einem Träger in Kontakt gebracht und dann das organische Lösungsmittel verdampft werden. Die In-Kontakt-Bringung kann z.B. so erfolgen, dass man in einem geeigneten Mischbehälter (z.B. Lödige-Mischer) den Träger vorlegt und dann die organische Lösung feinverteilt auf den Träger aufsprüht und dabei gleichzeitig das Lösungsmittel verdampft, wodurch der Träger mit den Verbindungen der Formel (I) beschichtet wird. Bevorzugt wird das Lösungsmittel unter vermindertem Druck verdampft, wobei die Temperatur des Trägers im Bereich von ca. 20° bis 80°C, bevorzugt in Bereich von 30° bis 70°C, liegt.

Als Träger für die In-Kontakt-Bringung mit den erfindungsgemäßen organischen Lösungen, die die Verbindungen der Formel (I) enthalten, kommen z.B. in Frage Silikate, Tonerde, Kaolin, Kieselerde, Talkum, Kreide, Metalloxide, Metallcarbonate, Kieselgur, Ruße und/oder Kieselsäure. Bevorzugt werden Kieselgur, Ruß und/oder Kieselsäure angewendet. Von den Rußen sind solche bevorzugt, die eine DBP-Zahl von >30 ml/100 g, bevorzugt >50 ml/100 g, aufweisen. Die Ruße können als Pulver oder in geperlter Form eingesetzt werden. Bevorzugt werden die Ruße in geperlter Form eingesetzt. Von den Kieselsäuren werden bevorzugt hochdisperse Typen eingesetzt. Hochdisperse Kieselsäuren werden hergestellt z.B. durch Fällung von Lösungen von Silikaten oder durch Flammhydrolyse von Siliciumhalogeniden und haben eine spezifische Oberfläche von 20 bis 400 m³/g (BET-Oberfläche), bevorzugt von 100 bis 250 m³/g, ganz besonders bevorzugt von 120 bis 200 m³/g, und eine Primärteilchengröße von 10 bis 400 nm. Die Kieselsäuren können als Pulver, als Granulat oder als Mikrogranulat eingesetzt werden. Bevorzugt wird Mikrogranulat eingesetzt.

Das Massenverhältnis von den einzusetzenden Trägern zu den auf die Träger aufzubringenden Verbindungen der Formel (I) beträgt ca. 7:3 bis 3:7, bevorzugt 6:4 bis 4:6.

Das erfindungsgemäße Lösungs-Verfahren, gekoppelt mit der hier beschriebenen Beschichtung von Trägern mit den Verbindungen der Formel (I), benötigt nicht die aufwändige Mahlung und gegebenenfalls Beölung der Verbindungen der Formel (I) für den Einsatz z.B. als Vulkanisationsmittel für Dien-Kautschuke. Die mit den Verbindungen der Formel (I) beschichteten Träger können unter Berücksichtigung des Wirkstoffgehaltes auf dem Träger für die Vulkanisation wie die Verbindungen der Formel (I) selbst verwendet werden.

### Beispiele

In den nachfolgenden Beispielen wurde der pH-Wert jeweils mit einer pH-Elektrode bestimmt.

### Beispiel 1

In einer mit Stickstoff gespülten 2 1-Vierhalskolbenrührapparatur mit Innenthermometer, Rückflusskühler mit Blasenzähler und pH-Elektrode wurden 99,6 g (0,25 mol) 1,6-Hexamethylen-bis-(Natriumthiosulfat)-Dihydrat (Duralink^{®} HTS, 98%iges Produkt der Firma Flexsys/Belgien), gelöst in 300 g entionisiertem Wasser, vorgelegt. Der Rührer wurde eingeschaltet. Man fügte 21,0 g (0,25 mol) Natriumhydrogencarbonat, 21,0 g (0,25 mol) wässrige Formaldehyd-Lösung (36-38 %ig) und 43,3 g Toluol (25 Gew.-Teile bezogen auf 100 Gew.-Teile der theoretisch zu erwartenden Ausbeute an 1,6-Bis(N,N-dibenzylthiocarbamoyldithio)hexan) zu. Man spülte den Gasraum des Reaktionsgefäßes nochmals kurz mit Stickstoff. Bei einer Reaktorinnentemperatur von 23°C wurde dann, nachdem das Natriumhydrogencarbonat aufgelöst war, mit dem Zulauf von Natriumdibenzyldithiocarbamat-Lösung (NaBEC-Lösung), die eine Temperatur von ca. 22°C aufwies, begonnen. Insgesamt wurden 864 g (0,5 mol) wässrige NaBEC-Lösung (17,1%ig) gleichmäßig während 0,5 h zugetropft, wobei die Reaktionstemperatur durch Kühlung weiterhin bei ca. 23°C gehalten wurde. Sodann rührte man bei dieser Temperatur noch 22 h nach. Unmittelbar vor Beginn des NaBEC-Zulaufs lag der pH-Wert bei ca. 8,4. Im Laufe des Zulaufs der NaBEC-Lösung stieg der pH-Wert sehr steil an und erreichte am Ende des NaBEC-Zulaufs einen Wert von ca. 11,1. Am Ende der Nachrührzeit lag der pH-Wert noch immer bei ca. 11,1. Der ausgefallene Feststoff konnte durch Nutschen sehr leicht isoliert werden. Der Feststoff auf der Nutsche wurde portionsweise mit insgesamt 31 entionisiertem Wasser gewaschen, was sehr leicht vonstatten ging. Die Trocknung des Produktes erfolgte bei ca. 50°C und ca. 150 mbar im Vakuumtrockenschrank bis zur Gewichtskonstanz. Die Ausbeute an 1,6-Bis(N,N-dibenzylthiocarbamoyldithio)hexan betrug ca. 96%, bezogen auf eingesetztes 1,6-Hexamethylen-bis(Natriumthiosulfat)-Dihydrat. Der Schmelzpunkt des erhaltenen Produktes lag bei ca. 93°C und der Gehalt (HPLC, externer Standard) bei ca. 99%. Der Glührückstand (750°C/2h) wurde zu ca. 0,02 Gew.-% ermittelt.

### Beispiel 2

Man arbeitete wie im Beispiel 1, setzte jedoch kein Natriumhydrogencarbonat ein und tropfte die NaBEC-Lösung (19,1 %ig; 773,3 g), die eine Temperatur von ca. 22°C aufwies, gleichmäßig innerhalb von 1 h zu. Unmittelbar vor Beginn des NaBEC-Zulaufs lag der pH-Wert bei ca. 8,6. Im Laufe des Zulaufs der NaBEC-Lösung stieg der pH-Wert sehr steil an und erreichte am Ende des NaBEC-Zulaufs einen Wert von ca. 12,5. Am Ende der Nachrührzeit lag der pH-Wert bei ca. 12,4. Das sich gebildete Reaktionsprodukt konnte sehr leicht durch Abnutschen isoliert und anschließend problemlos gewaschen werden. Die Ausbeute an 1,6-Bis(N,N-dibenzylthiocarbamoyldithio)hexan betrug ca. 94 %, bezogen auf eingesetztes 1,6-Hexamethylen-bis(Natriumthiosulfat)-Dihydrat. Der Gehalt (HPLC, externer Standard) lag bei ca. 98%, der Schmelzpunkt bei ca. 92°C. Der Glührückstand (750°C/2h) wurde zu ca. 0,05 Gew.-% bestimmt.

### Beispiel 3

Man arbeitete analog zum Beispiel 1. Von den vorzulegenden Einsatzstoffen wurden alle außer Natriumhydrogencarbonat im Kolben vorgelegt. Der pH-Wert dieser Vorlage wurde bei ca. 20°C mit 45 %iger Natronlauge auf ca. 13 eingestellt (Verbrauch an 45 %iger Natronlauge ca. 13,1 g). Sodann tropfte man die NaBEC-Lösung (19,1 %ig; 773,3 g), die eine Temperatur von ca. 22°C aufwies, gleichmäßig innerhalb 1 h zu. Während des NaBEC-Zulaufs und während der sich anschließenden Nachreaktion von 22 h wurde der pH-Wert der Reaktionsmischung durch Zugabe von 5 %iger Natronlauge mittels einer pH-Wert-gesteuerten Dosierpumpe auf pH 13 gehalten. Das sich gebildete Reaktionsprodukt konnte leicht durch Abnutschen isoliert und anschließend problemlos gewaschen werden. Die Ausbeute an 1,6-Bis(N,N-dibenzylthiocarbamoyldithio)hexan betrug ca. 94%, bezogen auf eingesetztes 1,6-Hexamethylen-bis(Natriumthiosulfat)-Dihydrat. Der Gehalt (HPLC, externer Standard) lag bei ca. 97 %, der Schmelzpunkt bei ca. 92°C.

### Beispiel 4

In einer mit Stickstoff gespülten 2 1-Vierhalskolbenrührapparatur mit Innenthermometer, Rückflusskühler mit Blasenzähler und pH-Elektrode wurden 136,5 g (0,55 mol) Natriumthiosulfatpentahydrat und 300 g entionisiertes Wasser vorgelegt. Der Rührer wurde eingeschaltet. Nachdem das Thiosulfat gelöst war, gab man 38,8 g (0,25 mol) 1,6-Dichlorhexan hinzu. Die schwach saure Mischung wurde mit 2,5%iger Natronlauge auf pH 7,2 ± 0,1 eingestellt. Man spülte das Reaktionsgefäß nochmals kurz mit Stickstoff und kochte dann die Mischung 9 h lang am Rückfluss, wobei während dieser Zeit der pH-Wert der Reaktionsmischung durch Zugabe von 2,5 %iger Natronlauge mittels einer Dosierpumpe auf 7,2 ± 0,1 gehalten wurde (Steuerung mittels pH-Elektrode). Nach jeweils 6 und 8 Stunden der Reaktion wurde der Blasenzähler vom Rückflusskühler kurz abgenommen. Sodann wurden in den Rückflusskühler von oben je ca. 5 ml vollentsalztes Wasser mit einer Spritzflasche eingespritzt um eventuell nicht zurückgetropftes 1,6-Dichlorhexan in den Kolben zurückzuspülen. Nach beendeter Reaktionszeit waren ca. 15 ml an 2,5 %iger Natronlauge zur pH-Wert-Steuerung verbraucht. Nach kurzem Abkühlen wurde dem Reaktionsgemisch eine kleine Probe zur Umsatzbestimmung von 1,6-Dichlorhexan durch Gaschromatographie (GC) entnommen. Die GC-Analyse mit internem Standard ergab einen Restgehalt an 1,6-Dichlorhexan von <10 ppm, was einem Umsatz an 1,6-Dichlorhexan von >99,9 % entspricht. Das auf Raumtemperatur abgekühlte Reaktionsgemisch wurde mit 21,0 g (0,25 mol) wässriger Formaldehyd-Lösung (36-38 %ig), 21,0 g (0,25 mol) Natriumhydrogencarbonat und 43,3 g Toluol (25 Gew.-Teile bezogen auf 100 Gew.-Teile der theoretisch zu erwartenden Ausbeute an 1,6-Bis(N,N-dibenzylthiocarbamoyldithio)hexan) versetzt. Der Gasraum des Reaktors wurde nochmals kurz mit Stickstoff gespült. Sobald das Hydrogencarbonat aufgelöst war, wurde bei einer Reaktorinnentemperatur von ca. 23°C mit dem Zulauf von 773,3 g (0,5 mol) Natriumdibenzyldithiocarbamat-Lösung (NaBEC-Lösung) (19,1%ig), die eine Temperatur von ca. 22°C aufwies, begonnen. Während des gleichmäßigen Zulaufs der NaBEC-Lösung über 1 h wurde die Reaktorinnentemperatur auf ca. 23°C gehalten. Nach Zulaufende rührte man bei ca. 23°C noch 22 h nach. Unmittelbar vor dem Beginn des NaBEC-Zulaufs lag der pH-Wert bei ca. 8,3. Mit Beginn des NaBEC-Zulaufs stieg der pH-Wert sehr steil an und erreichte am Ende des NaBEC-Zulaufs einen Wert von ca. 10,7. Am Ende der Nachrührzeit lag der pH-Wert ebenfalls bei ca. 10,7. Der ausgefallene Feststoff konnte durch Nutschen sehr leicht isoliert werden. Das Produkt wurde auf der Nutsche portionsweise mit insgesamt 21 vollentsalztem Wasser gewaschen, was sehr leicht vonstatten ging. Die Trocknung des Produktes erfolgte bei 50°C und ca. 150 mbar im Vakuumtrockenschrank bis zur Gewichtskonstanz. Die Ausbeute betrug ca. 91% der Theorie bezogen auf eingesetztes 1,6-Dichlorhexan. Der Gehalt an 1,6-Bis(N,N-dibenzylthiocarbamoyldithio)hexan wurde mittels HPLC (externer Standard) zu ca. 96% bestimmt. Der Schmelzpunkt lag bei ca. 92°C. Der Glührückstand (750°C/2h) des Produktes betrug ca. 0,2 Gew.-%.

### Beispiele 5 bis 7

Man arbeitete analog zum Beispiel 1. Es wurde jedoch die Toluolmengen, die Reaktionstemperatur und die Zulaufzeit variiert. Die Nachrührzeit nach Zulaufende betrug jeweils 5 h. Die Temperatur des Zulaufs an Natriumdibenzyldithiocarbamat-Lösung (NaBEC-Lösung) betrug jeweils ca. 22°C. Die erhaltenen Reaktionsprodukte konnten sehr leicht abgenutscht werden. Die sich anschließende Wäsche wurde portionsweise mit entionisiertem Wasser durchgeführt, was sehr leicht vonstatten ging.

**Tabelle 1**

| | | Beispiel 5 | Beispiel 6 | Beispiel 7 |
|---|---|---|---|---|
| Reaktionstemperatur | (°C) | 23 | 23 | 40 |
| NaBEC-Lösung | | | | |
| Zulaufzeit | (h) | 0,5 | 1 | 0,5 |
| Einsatzmenge | (g) | 815 | 901 | 815 |
| Konzentration | (%) | 18,1 | 16,4 | 18,1 |
| Toluolmenge | (g) | 26,0 | 87,0 | 17,3 |
| | (Gew.-Teile)¹⁾ | 15,0 | 50,2 | 10,0 |
| Ausbeute an 1,6-Bis-(N,N-dibenzylthiocarbamoyldithio)-hexan bezogen auf | | | | |
| Duralink^{®} HTS | (%) | 97 | 92 | 95 |
| Gehalt, HPLC²⁾ | (%) | 97 | 99 | 99 |

| | | | | |
|---|---|---|---|---|
| ¹⁾ bezogen auf 100 Gew.-Teile der theoretisch zu erwartenden Ausbeute an 1,6-Bis(N,N-dibenzylthiocarbamoyldithio)hexan. ²⁾ externer Standard | | | | |

### Beispiele 8 bis 10

Man arbeitete analog zum Beispiel 1, jedoch mit unterschiedlichen Lösungsmitteln und bei unterschiedlichen Reaktionstemperaturen. Der Zulauf der Natriumdibenzyldithiocarbamat-Lösung (NaBEC-Lösung) wies jeweils eine Temperatur von ca. 22°C auf. Die Reaktionsprodukte konnten sehr leicht isoliert und gewaschen werden.

**Tabelle 2**

| | | Beispiel 8 | Beispiel 9 | Beispiel 10 |
|---|---|---|---|---|
| Reaktionstemperatur | (°C) | 40 | 23 | 23 |
| Lösungsmittel | | n-Hexan | Chlorbenzol | Cyclohexan |
| Lösungsmittelmenge | (g) | 33 | 11,1 | 54,5 |
| | (Gew.-Teile)¹⁾ | 19,0 | 6,4 | 31,5 |
| NaBEC-Lösung | | | | |
| Zulaufzeit | (h) | 0,5 | 1 | 1 |
| Einsatzmenge | (g) | 815 | 1019 | 1019 |
| Konzentration | (%) | 18,1 | 14,5 | 14,5 |
| Ausbeute an 1,6-Bis-(N,N-dibenzylthiocarbamoyldithio)-hexan bezogen auf | | | | |
| Duralink^{®} HTS | (%) | 94 | 95 | 95 |
| Gehalt, HPLC²⁾ | (%) | 99 | 99 | 99 |
| Schmelzpunkt | (°C) | 94 | 93 | 93 |

| | | | | |
|---|---|---|---|---|
| ¹⁾ bezogen auf 100 Gew.-Teile der theoretisch zu erwartenden Ausbeute an Verbindung 1,6-Bis(N,N-dibenzylthiocarbamoyldithio)hexan ²⁾ externer Standard | | | | |

Im Beispiel 9 wurde das Reaktionsprodukt auf der Nutsche zunächst mit Methanol gewaschen, um das Chlorbenzol schneller zu verdrängen. Erst dann wurde mit entionisiertem Wasser gewaschen. In den Beispielen 8 und 10 wurden die Reaktionsprodukte auf der Nutsche nur mit entionisiertem Wasser gewaschen.

### Beispiele 11 und 12

Man arbeitete entsprechend dem Beispiel 1, jedoch wurde die Formaldehyd-Einsatzmenge variiert und die Nachrührzeit nach Zugabe der NaBEC-Lösung jeweils auf 1 h verkürzt. Der Zulauf der Natriumdibenzyldithiocarbamat-Lösung (NaBEC-Lösung) wies jeweils eine Temperatur von ca. 22°C auf. Die Reaktionsprodukte wurden auf der Nutsche portionsweise mit entionisiertem Wasser gewaschen, was sehr leicht vonstatten ging.

**Tabelle 3**

| | | Beispiel 11 | Beispiel 12 |
|---|---|---|---|
| Menge Formaldehydlösung (37-38%ig) | (g) | 21 | 84 |
| Formaldehyd bezogen | | | |
| auf Duralink^{®} HTS | (Mol%) | 105 | 420 |
| NaBEC-Lösung | | | |
| Zulaufzeit | (h) | 1 | 1 |
| Einsatzmenge | (g) | 890 | 890 |
| Konzentration | (%) | 16,6 | 16,6 |
| Ausbeute 1,6-Bis-(N,N-dibenzylthiocarbamoyldithio)-hexan bezogen auf | | | |
| Duralink^{®} HTS | (%) | 87 | 93 |
| Gehalt, HPLC¹⁾ | (%) | 99 | 99 |
| Schmelzpunkt | (°C) | 93 | 93 |

| | | | |
|---|---|---|---|
| ¹⁾ externer Standard | | | |

### Beispiel 13

In einer mit Stickstoff gespülten 4 1-Vierhalskolbenrührapparatur mit Innenthermometer, Rückflusskühler und pH-Elektrode wurden 240 g entionisiertes Wasser, 79,7 g (0,20 mol) 1,6-Hexamethylen-bis(Natriumthiosulfat)-Dihydrat (Duralink^{®} HTS 98%ig von Firma Flexsys/Belgien), 16,8 g (0,20 mol) Natriumhydrogencarbonat, 16,8 g (0,20 mol) wässriger Formaldehyd-Lösung (35,8%ig) und 779 g Toluol (562 Gew.-Teile bezogen auf 100 Gew.-Teile der theoretisch zu erwartenden Ausbeute an 1,6-Bis(N,N-dibenzylthiocarbamoyldithio)hexan) vorgelegt. Man spülte das Reaktionsgfäß nochmals kurz mit Stickstoff und heizte dann auf 50°C auf. Bei einer Reaktorinnentemperatur von 50°C wurde dann mit dem Zulauf von Natriumdibenzyldithiocarbamat (NaBEC)-Lösung, die eine Temperatur von ca. 22°C aufwies, begonnen. Insgesamt wurden 721g (0,4 mol) wässrige NaBEC-Lösung (16,4%ig) gleichmäßig während 1 h zugetropft, wobei die Reaktionstemperatur weiterhin bei 50°C gehalten wurde. Sodann rührte man bei dieser Temperatur noch 5 h nach. Unmittelbar vor Beginn des NaBEC-Zulaufs lag der pH-Wert bei ca. 8,5. Mit Beginn des NaBEC-Zulaufs stieg der pH-Wert steil an und erreichte am Ende des NaBEC-Zulaufs einen Wert von ca. 10,8. Am Ende der Nachrührzeit lag der pH-Wert bei 50°C noch immer bei ca. 10,8 und alles gebildete Reaktionsprodukt war vollständig im Toluol gelöst. Nach der Phasentrennung wurde die organische Phase viermal mit jeweils ca. 50 ml entionisiertem Wasser gewaschen und dann das Toluol am Rotationsverdampfer im Vakuum bei einer Badtemperatur von ca. 50°C eingedampft. Das erhaltene zähe Öl konnte nur schwer zur Kristallisation gebracht werden. Die schließlich gewonnenen Kristalle wurden im Vakuumtrockenschrank bei ca. 50°C und ca. 150 mbar bis zur Gewichtskonstanz getrocknet. Die Ausbeute betrug ca. 95% bezogen auf eingesetztes 1,6-Hexamethylen-bis(Natriumthiosulfat)-Dihydrat. Der Schmelzpunkt des Produktes wurde zu ca. 92°C bestimmt, der Gehalt an 1,6-Bis(N,N-dibenzylthiocarbamoyldithio)hexan zu ca. 98 % (HPLC, externer Standard) und der Glührückstand (750°C/2h) zu 0,01 Gew.-%.

### Beispiele 14 und 15

Man arbeitete wie im Beispiel 13, führte die Umsetzung jedoch bei einer Reaktionstemperatur von 23° und 70°C durch. Der Zulauf der Natriumdibenzyldithiocarbamat-Lösung (NaBEC-Lösung) wies jeweils eine Temperatur von ca. 22°C auf.

**Tabelle 4**

| | | Beispiel 14 | Beispiel 15 |
|---|---|---|---|
| Reaktionstemperatur | (°C) | 23 | 70 |
| NaBEC-Lösung | | | |
| Einsatzmenge | (g) | 720,5 | 695,1 |
| Konzentration | (%) | 16,4 | 17,0 |
| Ausbeute an 1,6-Bis-(N,N-dibenzylthiocarbamoyldithio)-hexan bezogen auf | | | |
| Duralink^{®} HTS | (%) | 97 | 89 |
| Gehalt, HPLC¹⁾ | (%) | 99 | 97 |
| Schmelzpunkt | (°C) | 93 | 92 |
| ¹⁾ externer Standard | | | |

Bemerkung: In beiden Beispielen war bei der jeweiligen Reaktionstemperatur am Ende der Umsetzung alles gebildete Reaktionsprodukt im Toluol gelöst. Das nach Aufarbeitung jeweils erhaltene zähe Öl kristallisierte nur sehr langsam.

### Beispiel 16

Man legte 50 g Kieselsäure Zeosil^{®} 1165 MP (Produkt der Firma Rhodia Silica Systems) in einem 1 1-Rundkolben vor und versetzte dann in kleinen Portionen mit insgesamt 337,8 g einer ca. 14,8 %igen Lösung von 1,6-Bis(N,N-dibenzylthiocarbamoyldithio)hexan in Toluol, wobei man nach jeder Portionszugabe das Lösungsmittel am Rotationsverdampfer bei Wasserstrahlpumpenvakuum und einer Badtemperatur von maximal 75°C verdampfte, ohne dass dabei die Produkttemperatur auf über 50°C anstieg. Anschließend wurde die beschichtete Kieselsäure im Vakuumtrockenschrank bei ca. 50°C und ca. 150 mbar bis zur Gewichtskonstanz getrocknet. Es wurde ein beschichtetes, praktisch klumpenfreies, freifließendes, praktisch nicht staubendes Mikrogranulat erhalten, dessen Gehalt an 1,6-Bis(N,N-dibenzylthiocarbamoyldithio)hexan ca. 48 % betrug (HPLC, externer Standard).

### Beispiele 17 und 18 (Vergleichsbeispiele analog DE-A 22 56 511, Seite 39)

In einer mit Stickstoff gespülten 2 1-Vierhalskolbenrührapparatur mit Innenthermometer und Rückflusskühler wurden 300 g entionisiertes Wasser und 99,6 g (0,25 mol) 1,6-Hexamethylenbis(Natriumthiosulfat)-Dihydrat (Duralink^{®} HTS, 98%iges Produkt der Firma Flexsys/Belgien) vorgelegt. Man fügte 102,1 g (0,75 mol) Natriumacetattrihydrat und 48,0 g (0,583 mol) wässrige Formaldehyd-Lösung (36,5%ig) zu. Nachdem sich alles Natriumacetattrihydrat gelöst hatte, wurden bei einer Reaktorinnentemperatur von ca. 23°C 971,7 g (0,5 mol) wässrige Natriumdibenzyldithiocarbamat-Lösung (NaBEC-Lösung) (15,2%ig), die eine Temperatur von ca. 22°C aufwies, gleichmäßig während 1 h zugetropft. Unmittelbar vor Beginn des NaBEC-Zulaufs lag im Beispiel 17 der pH-Wert bei 8,7, am Ende des Zulaufs bei 12,4. Im Falle des Beispiels 18 lag der pH-Wert unmittelbar vor Beginn des Zulaufs bei 9,4 und am Ende des Zulaufs bei 12,4. Man rührte im Beispiel 17 bei ca. 22°C noch 5 h nach bzw. 22 h im Beispiel 18. Der jeweils äußerst feine, weiße Feststoffe wurden durch Filtration isoliert und portionsweise mit jeweils insgesamt 41 entionisiertem Wasser gründlich gewaschen. Aufgrund der hohen Feinheit der Kristalle war die Isolierung und die Wäsche der Produkte sehr langwierig und schwierig. Das gewaschene Reaktionsprodukt wurde bei ca. 50°C und ca. 150 mbar im Vakuumtrockenschrank getrocknet.

**Tabelle 5**

| | | Beispiel 17 (Vergleich) | Beispiel 18 (Vergleich) |
|---|---|---|---|
| Nachrührzeit | (h) | 5 | 22 |
| Ausbeute an 1,6-Bis-(N,N-dibenzylthiocarbamoyldithio)-hexan bezogen auf | | | |
| Duralink^{®} HTS | (%) | 82 | 86 |
| Gehalt, HPLC¹⁾ | (%) | 93 | 97 |
| Schmelzpunkt | (°C) | 85 | 89 |
| Glührückstand (750°C/2h) | (Gew.-%) | 0,3 | 0,3 |

| | | | |
|---|---|---|---|
| ¹⁾ externer Standard | | | |

Bewertung: Es ist festzustellen, dass man nach Stand der Technik eine niedrigere Ausbeute, einen niedrigeren Wirkstoffgehalt und einen niedrigeren Schmelzpunkt im Vergleich zu dem erfindungsgemäßen Beispiel 1 erhält.

### Beispiele 19 und 20 (Vergleichsbeispiele analog EP-A 432 417, Seite 4, Zeile 50ff)

Es wurde analog dem Beispiel 1 gearbeitet, jedoch ohne Anwendung der erfindungsgemäßen Maßnahmen, d.h. ohne Einsatz von Formaldehyd, Natriumhydrogencarbonat und Toluol. Im Beispiel 19 lag der pH-Wert unmittelbar vor Zulaufbeginn der NaBEC-Lösung bei ca. 8,6, bei Zulaufende bei ca. 9,3 und am Ende der Nachrührzeit von 5 h bei ca. 9,7. Im Beispiel 20 lag der pH-Wert unmittelbar vor Zulaufbeginn der NaBEC-Lösung bei ca. 8,7, bei Zulaufende bei ca. 9,5 und am Ende der Nachrührzeit von 22 h bei ca. 9,9. Das in den Beispielen 19 und 20 erhaltene Reaktionsprodukt war aufgrund der feinen Kristalle sehr schwierig zu waschen und nahm sehr lange Zeit in Anspruch.

**Tabelle 6**

| | | Beispiel 19 | Beispiel 20 |
|---|---|---|---|
| | | (Vergleich) | (Vergleich) |
| NaBEC-Lösung | | | |
| Zulaufzeit | (h) | 0,5 | 0,5 |
| Einsatzmenge | (g) | 777,3 | 777,3 |
| Konzentration | (%) | 19,1 | 19,1 |
| Nachrührzeit | (h) | 5 | 22 |
| Ausbeute an 1,6-Bis-(N,N-dibenzylthiocarbamoyldithio)-hexan bezogen auf | | | |
| Duralink^{®} HTS | (%) | 74 | 78 |
| Gehalt, HPLC¹⁾ | (%) | 96 | 91 |
| Schmelzpunkt | (°C) | 81-84 | 80-90 |
| Glührückstand (750°C/2h) | (Gew.-%) | 0,15 | 0,98 |

| | | | |
|---|---|---|---|
| ¹⁾ externer Standard | | | |

Bewertung: Nach Stand der Technik erhält man sehr feine, sehr schwierig und nur sehr langsam waschbare Produkte in niedriger Ausbeute. Die niedrige Ausbeute, der niedrige Wirkstoffgehalt und der breite Schmelzbereich des Produktes im Beispiel 20 wird im direkten Vergleich mit den erfindungsgemäßen Beispielen 1 und 2 besonders deutlich.

## Patentansprüche

1. Verfahren zur Herstellung der Verbindungen der Formel (I)
R¹R² N-(C=S)-S-S-(CH₂)ₙ-S-S-(C=S)-N R¹R² (I),
worin die Reste R¹ und R² unabhängig voneinander Wasserstoff, Alkyl, Cycloalkyl, Aralkyl, Phenyl oder substituiertes Phenyl oder Alkylaryl, bedeuten oder zusammen mit dem jeweiligen Stickstoffatom einen heterocyclischen Ring mit 4 bis 8 Kohlenstoffatomen bilden und n eine ganze Zahl von 2 bis 8 darstellt,
**dadurch gekennzeichnet, dass** man die Verbindungen der Formel (II)
[R¹R²N-(C=S)-S]_{z}Me¹ (II),
worin die Reste R¹ und R² die gleiche Bedeutung wie in der Formel (I) haben und z = 1 ist, wenn Me¹ ein Alkalimetall- oder ein Ammoniumion bedeutet und z = 2 ist, wenn Me¹ ein Erdalkalimetallion bedeutet,
mit den Verbindungen der Formel (III)
Me²O₃SS-(CH₂)ₙ-SSO₃Me³ (III),
worin Me² und Me³ gleich oder verschieden sind und einwertige Metallionen oder Ammoniumionen bedeuten und n die gleiche Bedeutung wie in der Formel (I) hat,
in Gegenwart von Wasser, Carbonylverbindungen und organischen Lösungsmitteln in einem pH-Wert-Bereich von 7 bis 14 bei einer Reaktionstemperatur von 0° bis 90°C bis zu einer Ausbeute von mindestens 80 % der Theorie, bezogen auf die Salze der Formel (III), umsetzt, wobei die Verbindungen der Formel (II) in Mengen von 180 bis 250 Mol-%, bezogen auf die Mole an eingesetzten Verbindungen der Formel (III), die Carbonylverbindungen in Mengen von 5 bis 600 Mol%, bezogen auf die Mole an eingesetzten Verbindungen der Formel (III), und die organischen Lösungsmittel in Mengen von 2 bis 100 000 Gew.-Teilen, bezogen auf 100 Gew.-Teile der theoretisch zu erwartenden Menge an Verbindungen der Formel (I), eingesetzt werden.

2. Verfahren nach Anspruch 1, **dadurch gekennzeichnet, dass** die organischen Lösungsmittel in Mengen von 2 bis 100 Gew.-Teilen, bezogen auf 100 Gew.-Teile der theoretisch zu erwartenden Menge an Verbindungen der Formel (I), eingesetzt werden und eine Reaktionstemperatur im Bereich von 0° bis 60°C angewendet wird, wobei am Ende der Reaktion die in den organischen Lösungsmitteln gelöste Menge an den Verbindungen der Formel (I) höchstens 15 Gew.-Teile, bezogen auf 100 Gew.-Teile der durch Fest-Flüssig-Trennung isolierbaren Verbindungen der Formel (I), beträgt.

3. Verfahren nach Anspruch 1, **dadurch gekennzeichnet, dass** die organischen Lösungsmittel in Mengen von 105 bis 100 000 Gew.-Teilen, bezogen auf 100 Gew.-Teile der theoretisch zu erwartenden Menge an Verbindungen der Formel (I), eingesetzt werden und eine Reaktionstemperatur im Bereich von 0° bis 90°C angewendet wird, wobei am Ende der Reaktion die Verbindungen der Formel (I) in den organischen Lösungsmitteln vollständig gelöst sind.

4. Verfahren nach Anspruch 3, **dadurch gekennzeichnet, dass** man die erhaltene Reaktionslösung mit einem Träger in Kontakt bringt und dann das organische Lösungsmittel verdampft.

## Claims

1. Process for preparing compounds of the formula (I) R
¹R² N-(C=S)-S-S-(CH₂)ₙ -S-S(C=S)-NR¹R² (I),
in which the R¹ and R² radicals are each independently hydrogen, alkyl, cycloalkyl, aralkyl, phenyl or substituted phenyl or Alkylaryl, or, together with the particular nitrogen atom, form a heterocyclic ring having 4 to 8 carbon atoms and n is an integer from 2 to 8,
**characterized in that** the compounds of the formula (II)
[R¹R² N-(C=S)-S]_{z}Me¹ (II)
in which the R¹ and R² radicals are each as defined in the formula (I) and z = 1 when Me¹ is an alkali metal or ammonium ion and z = 2 when Me¹ is an alkaline earth metal ion,
are reacted with the compounds of the formula (III)
Me² O₃S S-(CH₂)ₙ-S SO₃ Me³ (III)
where Me² and Me³ are the same or different and are each monovalent metal ions or ammonium ions and n is as defined in the formula (I)
in the presence of water, carbonyl compounds and organic solvents in a pH range of 7 to 14 at a reaction temperature of 0° to 90°C up to a yield of at least 80% of theory based on the salts of the formula (III), the compounds of the formula (II) being used in amounts of 180 to 250 mol%, based on the moles of compounds of the formula (III) used, the carbonyl compounds in amounts of 5 to 600 mol%, based on the moles of compounds of the formula (III) used, and the organic solvents in amounts of 2 to 100 000 parts by weight based on 100 parts by weight of the theoretically expected amount of compounds of the formula (I).

2. Process according to Claim 1, **characterized in that** the organic solvents are used in amounts of 2 to 100 parts by weight based on 100 parts by weight of the theoretically expected amount of compounds of the formula (I) and a reaction temperature in the range of 0 to 60°C is employed, the amount of the compounds of the formula (I) dissolved in the organic solvents at the end of the reaction being at most 15 parts by weight based on 100 parts by weight of the compounds of the formula (I) isolable by solid-liquid separation.

3. Process according to Claim 1, **characterized in that** the organic solvents are used in amounts of 105 to 100 000 parts by weight based on 100 parts by weight of the theoretically expected amount of compounds of the formula (I) and a reaction temperature in the range of 0° to 90°C is employed, the compounds of the formula (I) being dissolved fully in the organic solvents at the end of the reaction.

4. Process according to Claim 3, **characterized in that** the resulting reaction solution is contacted with a support and then the organic solvent is evaporated.

## Revendications

1. Procédé de fabrication de composés de formule (I)
**R¹R²N-(C=S)-S-S-(CH₂)ₙ-S-S-(C=S)-NR¹R²** **(I),**
dans laquelle les radicaux R¹ et R² signifient indépendamment l'un de l'autre hydrogène, alkyle, cycloalkyle, aralkyle, phényle ou phényle substitué ou alkylaryle, ou forment ensemble avec l'atome d'azote respectif un cycle hétérocyclique de 4 à 8 atomes de carbone, et n représente un nombre entier de 2 à 8, **caractérisé en ce que** les composés de formule (II)
**[R¹R²N-(C=S)-S]₂Me¹** **(II),**
dans laquelle les radicaux R¹ et R² ont la même signification que dans la formule (I) et z = 1 lorsque Me¹ signifie un ion de métal alcalin ou d'ammonium, et z = 2 lorsque Me¹ signifie un ion de métal alcalinoterreux,
sont mis en réaction avec les composés de formule (III)
**Me²O₃SS-(CH₂)ₙ-SSO₃Me³** **(III),**
dans laquelle Me² et Me³ sont identiques ou différents et signifient des ions métalliques monovalents ou des ions ammonium, et n a la même signification que dans la formule (I),
en présence d'eau, de composés de carbonyle et de solvants organiques dans une plage de pH allant de 7 à 14 à une température de réaction de 0 °C à 90 °C jusqu'à un rendement d'au moins 80 % de la théorie, par rapport aux sels de formule (III), les composés de formule (II) étant utilisés en quantités de 180 à 250 % en moles, par rapport aux moles de composés de formule (III) utilisés, les composés de carbonyle en quantités de 5 à 600 % en moles, par rapport aux moles de composés de formule (III) utilisés, et les solvants organiques en quantités de 2 à 100 000 parties en poids, par rapport à 100 parties en poids de la quantité de composés de formule (I) attendue en théorie.

2. Procédé selon la revendication 1, **caractérisé en ce que** les solvants organiques sont utilisés en quantités de 2 à 100 parties en poids, par rapport à 100 parties en poids de la quantité de composés de formule (I) attendue en théorie, et une température de réaction dans la plage allant de 0° à 60 °C est utilisée, la quantité de composés de formule (I) dissoute dans les solvants organiques à la fin de la réaction étant d'au plus 15 parties en poids, par rapport à 100 parties en poids des composés de formule (I) pouvant être isolés par séparation solide-liquide.

3. Procédé selon la revendication 1, **caractérisé en ce que** les solvants organiques sont utilisés en quantités de 105 à 100 000 parties en poids, par rapport à 100 parties en poids de la quantité de composés de formule (I) attendue en théorie, et une température de réaction dans la plage allant de 0° à 90 °C est utilisée, les composés de formule (I) étant dissous en totalité dans les solvants organiques à la fin de la réaction.

4. Procédé selon la revendication 3, **caractérisé en ce que** la solution réactionnelle obtenue est mise en contact avec un support, puis le solvant organique est évaporé.
